# EUROPEAN PATENT APPLICATION

(11) **EP 4 599 858 A1**
(43) Date of publication of application: **13.08.2025**
(21) Application number: 23874545.9
(22) Date of filing: 21.08.2023
(51) Int. Cl.: A61L 31/02, A61M 25/09

(54) **MEDICAL WIRE MATERIAL AND GUIDE WIRE**

(30) Priority: 05.10.2022 JP 2022160751
(71) Applicant: ASAHI INTECC CO., LTD., Seto-shi, Aichi, 489-0071 (JP)
(72) Inventor: KAMAKURA, Nagisa, Seto-shi, Aichi 489-0071 (JP); SATO, Kazufumi, Seto-shi, Aichi 489-0071 (JP); NAKADA, Masakazu, Seto-shi, Aichi 489-0071 (JP); TSUCHIYAMA, Toshihiro, Fukuoka-shi, Fukuoka 819-0395 (JP); MASUMURA, Takuro, Fukuoka-shi, Fukuoka 819-0395 (JP)
(74) Representative: Winter, Brandl - Partnerschaft mbB
(86) International application number: PCT/JP2023/030000
(87) International publication number: WO 2024/075413

(57) **Abstract**

An object of the disclosure is to provide a medical wire material and a guide wire with excellent resilience.

A medical wire material is consisting of stainless steel, in which a shape of a transverse section of the wire material is a circle having a diameter of d mm, and, when hardness of the transverse section of the wire material is measured by the Nanoindentation method, an average value of hardness of an outer peripheral portion constituted of a region surrounded by an outer peripheral edge and a circle having a distance from the outer peripheral edge is d/17 mm is equal to or greater than 8.0 GPa.

## Description

### [Technical Field]

The disclosure relates to a medical wire material and a guide wire.

### [Background Art]

When treating a stenosis that occurs in blood vessels such as coronary arteries surrounding the heart, or when treating a region where the blood vessel is completely occluded due to the progress of calcification (for example, chronic total occlusion: CTO), a guide wire for guiding a treatment instrument such as a balloon catheter is inserted into the blood vessel, prior to the treatment instrument.

For example, Patent Literature 1 proposes a guide wire made of SUS304. Patent Literature 2 discloses a wire having an average of Vickers hardness at eight specific points in a cross section of equal to or greater than 670 and equal to or less than 770, for the purpose of providing a wire for a medical treatment tool having excellent fatigue resistance.

### [Citation List]

### [Patent Literature]

[Patent Literature 1] JP 2009-172229 A
[Patent Literature 2] JP 6596470 B2

### [Summary of Invention]

### [Technical Problem]

Now, regarding the above guide wire, it is known that there is a correlation between the operability of the guide wire and the ability to restore its original shape (resilience) after bending etc. However, no guide wire with excellent resilience has been proposed.

An object of the disclosure is to provide a medical wire material and a guide wire with excellent resilience.

### [Solution to Problem]

In order to solve the above problem, a medical wire material according to an aspect of the disclosure is a medical wire material consisting of stainless steel, in which a shape of a transverse section of the wire material is a circle having a diameter of d mm, and, when hardness of the transverse section of the wire material is measured by the Nanoindentation method, an average value of hardness of an outer peripheral portion constituted of a region surrounded by an outer peripheral edge and a circle having a distance from the outer peripheral edge is d/17 mm is equal to or greater than 8.0 GPa.

The stainless steel may be stable austenitic stainless steel having a temperature Md₃₀ that is equal to or lower than -50 degrees Celsius, the temperature Md₃₀ being the temperature at which strain-induced martensite of 50% occurs when a strain of 30% given by an expression of Angel is applied.

The stable austenitic stainless steel may be stainless steel conforming to ASTM F2581.

When the hardness of the transverse section of the wire material is measured by the Nanoindentation method, a difference between an average value of hardness of an entirety of the transverse section of the wire material and an average value of hardness of the outer peripheral portion may be equal to or less than 0.7 GPa.

The average value of the hardness of an entirety of the transverse section of the wire material may be equal to or greater than 8.9 GPa.

A guide wire according to one aspect of the disclosure includes the above-described medical wire material.

### [Advantageous Effects of Invention]

The disclosure can provide a medical wire material and a guide wire with excellent resilience and straightness.

### [Brief Description of Drawings]

FIG. 1 illustrates a schematic cross-sectional view of a guide wire according to an embodiment of the disclosure.
FIG. 2 is a schematic diagram illustrating a result of measuring a transverse section of a wire material by the Nanoindentation method.
FIG. 3 is a schematic diagram of a jig to be used for resilience evaluation.
FIG. 4 is an explanatory view of resilience evaluation.
FIG. 5 is an explanatory view of a method for measuring a residual angle.
FIG. 6 is a diagram illustrating only dots of an outer peripheral portion among all dots of the transverse section of the wire material illustrated in FIG. 2.
FIG. 7 is an explanatory view of wave height indicating straightness.

### [Description of Embodiments]

As a result of intensive study by the present inventors for a medical wire material excellent in resilience, it has been found that a medical wire material excellent in resilience can be achieved when hardness of an outer peripheral portion of a transverse section of the wire material is within a specific range. That is, a medical wire material of the disclosure is a medical wire material made of stainless steel, in which a shape of a transverse section of the wire material is a circle having a diameter of d mm, and, when hardness of the transverse section of the wire material is measured by the Nanoindentation method, an average value of hardness of an outer peripheral portion constituted of a region surrounded by an outer peripheral edge and a circle having a distance from the outer peripheral edge is d/17 mm is equal to or greater than 8.0 GPa.

The reason why the resilience is improved by the configuration described above is unclear. However, it is presumed that, when the wire material is bent, a strain amount of the outer peripheral portion of the wire material due to bending becomes larger than a strain amount of the inside of the wire material due to bending, and therefore, even when hardness of the inside is high, if hardness of the outer peripheral portion is low, the outer peripheral portion having a large strain amount is easy to deform, but the deformation is made to be difficult by the high hardness of the outer peripheral portion, which improves resilience.

The medical wire material of the disclosure has a shape of the transverse section of the wire material being a circle having a diameter of d mm. It is sufficient that the diameter d of the circle is, but not limited to, 0.02 to 2.00 mm. In a case of use in the guide wire as described later, d is particularly preferably, 0.10 to 1.00 mm, and preferably, 0.20 to 0.50 mm. Note that the transverse section is not necessarily a circle in the entire length of the wire material. For example, it is sufficient that transverse sections of a part are circles, and transverse sections of other part may not be circles. When transverse sections of a part are circles, it is sufficient that an average value of hardness of the outer peripheral portion is within the range in a portion where the transverse section is a circle.

In the medical wire material of the disclosure, when hardness of the transverse section of the wire material is measured by the Nanoindentation method, an average value of hardness of an outer peripheral portion constituted of an outer peripheral edge and a circle having a distance from the outer peripheral edge is d/17 mm is equal to or greater than 8.0 GPa. In view of further improving resilience and rotation operability, the average value of hardness of the outer peripheral portion is preferably, equal to or greater than 8.4 GPa, more preferably, equal to or greater than 8.9 GPa, and further more preferably, equal to or greater than 9.0 GPa. Although the upper limit value is not limited, for example, in view of fragility, the average value of hardness of the entire transverse section is preferably, equal to or less than 12.0 GPa. A specific method of calculating the average value of hardness of the outer peripheral portion are described below.

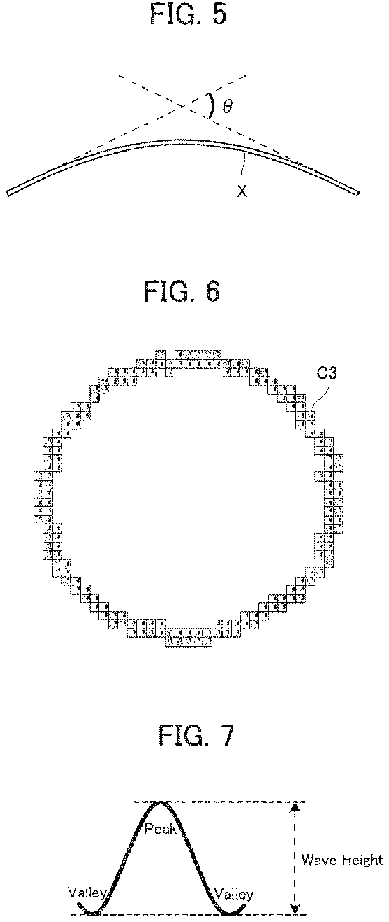

Herein, the hardness measurement by the Nanoindentation method provides a value obtained by conducting a measurement under the conditions of the maximum indentation load of 30 mN and an indentation interval of equal to or greater than 0.01 mm and less than 0.02 mm on the basis of ISO 14577 "instrumented indentation test". For example, an indentation interval X can be determined by setting n such that [diameter of wire material (mm)/(17 × n)] is equal to or greater than 0.01 mm and less than 0.02 mm. Specifically, in a case of measuring a wire material having a diameter of 0.34 mm, hardness of the outer peripheral portion can be obtained by setting the indentation interval by the Nanoindentation to 0.01 mm and calculating an average value of hardness for an amount of two dots in the outside (0.02 mm) as shown in FIG. 6. That is, hardness of the outer peripheral portion in this case can be obtained as an average value for an amount of two dots in the outside when measurement is conducted with the indentation interval of the Nanoindentation being [diameter of wire material (mm)/(17*2)].

In view of resilience and fragility, in the medical wire material of the disclosure, when hardness of the transverse section of the wire material is measured by the Nanoindentation method, it is preferable that a difference between an average value of hardness of the entire transverse section and an average value of hardness of an outer peripheral portion constituted of an outer peripheral edge and a circle having a distance from the outer peripheral edge is d/17 mm is equal to or less than 0.7 GPa. The difference is preferably, equal to or less than 0.6 GPa, more preferably, equal to or less than 0.5 GPa, and further more preferably, equal to or less than 0.4 GPa.

It is preferable that, in the medical wire material of the disclosure, an average value of hardness of the entire transverse section is equal to or greater than 8.0 GPa. Since resilience can be further improved, the average value of hardness of the entire transverse section is preferably, equal to or greater than 8.6 GPa, more preferably, equal to or greater than 8.9 GPa, and further more preferably, equal to or greater than 9.4 GPa. Although the upper limit value is not limited, for example, in view of fragility, the average value of hardness of the entire transverse section is preferably, equal to or less than 12.0 GPa. The average value of hardness of the entire transverse section may be a value greater than the average value of hardness of the outer peripheral portion.

The residual angle of the medical wire material of the disclosure is preferably, equal to or less than 12.0°, more preferably, equal to or less than 9.5°, and particularly preferably, equal to or less than 7.0.

In the medical wire material of the disclosure, the wave height indicating straightness is preferably, equal to or less than 1.0 mm, and more preferably, equal to or less than 0.5 mm. As shown in FIG. 7, the wave height can be obtained by drawing a straight line from a valley to a valley of one wave of the wire material, and measuring the height of a peak from the straight line. When the wire material has a plurality of waves, the wave height indicates the largest height of the wave.

It is preferable that the medical wire material of the disclosure has a tensile strength of equal to or greater than 2800 N/mm². Since the resilience can be further improved, the tensile strength is more preferably, equal to or greater than 2900 N/mm², and more preferably, equal to or greater than 3000 N/mm², and particularly preferably, equal to or greater than 3100 N/mm². The upper limit of the tensile strength of the wire material is generally, but not particularly limited to, equal to or less than 3500 N/mm². The tensile strength of the wire material can be measured by a general tensile test of a metal wire material (for example, a tensile test conforming to JIS Z 2241 "metal material tensile testing method").

The medical wire material of the disclosure can be manufactured by applying a wire drawing method to a steel material (base material) of stable austenitic stainless steel having a temperature Md₃₀ at which strain-induced martensite of 50% occurs when strain of 30% given by an expression of Angel is applied is equal to or lower than -50 degrees Celsius, and then performing specific straightening processing as a combination. In general, examples of straightening processing include a processing method of straightening by plastic deformation by rotary straightener, wire straightener, stretcher, or the like, and a processing method of straightening by applying heat such as tension annealing. As a result of intensive study by the present inventors, it is found that, in the stable austenitic stainless steel described above, hardness of the outer peripheral portion can be increased by straightening by plastic deformation and then straightening by heating, which can achieve a medical wire material having excellent resilience. It is found that, through the processes described above, the difference between the average hardness of the transverse section and the hardness of the outer peripheral portion can be reduced, and a wire material also having preferable straightness can be obtained.

For example, even in a case of using the stable austenitic stainless steel described above, when straightening is performed only by plastic deformation as Comparative Example 1 described later, the resilience is poor. It is found that the resilience can be also achieved as in Examples 1 to 5 described later by performing straightening by plastic deformation and then performing tension annealing by heat. In addition, regarding the material, it is found that, in the metastable austenitic stainless steel such as SUS304 in which strain-induced martensite occurs, as Comparative Example 2 described later, even when straightening by plastic deformation is performed and then tension annealing processing is performed, excellent resilience cannot be obtained.

As described above, the medical wire material of the disclosed embodiments can be first obtained by using a specific material and applying specific processing.

The medical wire material of the disclosure is constituted of stainless steel, preferably, stable austenitic stainless steel having a temperature Md₃₀ at which strain-induced martensite of 50% occurs when strain of 30% given by an expression of Angel as below is applied is equal to or lower than -50 degrees Celsius. Md30 = 551-462×(C+N)-9.2×Si-8.1×Mn-13.7×Cr-9.5Ni-18.5×Mo ... Expression of Angel Here, C, N, Si, Mn, Cr, Ni, Cu, and Mo are each element amount (mass%). As the value of Md₃₀ of the material is smaller, the austenite is more stable.

Since effects of solid-solution strengthening and strain aging are enhanced, it is particularly preferable that the stainless steel is high-nitrogen austenitic stainless steel having content of nitrogen (N) is equal to or greater than 0.1 mass%. The content of nitrogen is more preferably, equal to or greater than 0.2 mass%, and further more preferably, equal to or greater than 0.4 mass%. Since excellent breaking resistance can be achieved, the content of nitrogen is preferably, equal to or less than 1.0 mass%, and more preferably, equal to or less than 0.8 mass%.

Examples of the stainless steel described above include stainless steel conforming to ASTM F2581 (C: 0.15 to 0.25 mass%, Mn: 9.50 to 12.50 mass%, P: 0.020 mass% Max, S: 0.010 mass% Max, Si: 0.20 to 0.60 mass%, Cr: 16.5 to 18.0 mass%, Ni: 0.05 mass% Max, Mo: 2.70 to 3.70 mass%, N: 0.45 to 0.55 mass%, Cu: 0.25 mass% Max, Fe: Bal.), and ASTM F138, F1314, F1586, and F2229. Since a wire material having particularly excellent resilience can be obtained, the stainless steel conforming to ASTM F2581 is preferable.

The wire drawing is not particularly limited as long as it can continuously reduce the wire diameter of a steel material, and may be either a process using dies or a process using rolls. The area reduction rate of a wire material in processing ranges from preferably 80% to 97%, for example. Here, the area reduction rate is defined by (1-r1²/r0²)×100. Wherein r0 is the radius of a base material (wire material before processing), and r1 is the radius of a wire drawing material (wire material after processing).

The straightening processing of straightening by plastic deformation is not particularly limited as long as the processing is capable of correcting the wire material to be straight, and examples of such processing include processing by roller leveler, wire straightener, tension leveler, and stretcher, and these can be performed individually or in combination with each other. It is sufficient that the straightening processing by plastic deformation is performed at a normal temperature or a temperature that does not largely affect a metal, and it is preferable that the straightening processing is performed at a temperature of 0 degrees Celsius to 100 degrees Celsius.

The tension annealing processing is performed by heating a wire material while applying tensile force (tension) to the wire material. It is preferable that the tensile force is 10 to 40% of a tensile strength and a heating temperature is 300 to 700 degrees Celsius. In addition, it is more preferable that the tensile force is 20 to 30% and the heating temperature is 500 to 650 degrees Celsius.

Hereinafter, one embodiment of the guide wire of the disclosure is described with reference to drawings. However, the disclosure is not limited to only the embodiments illustrated in the drawings.

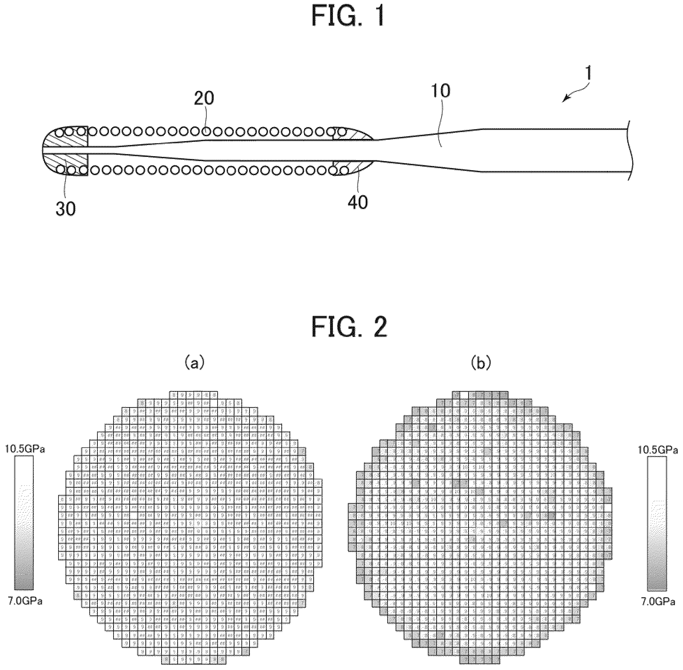

FIG. 1 is a schematic cross-sectional view of a guide wire 1 according to an embodiment of the disclosure.

As shown in FIG. 1, the guide wire 1 includes a core shaft 10, a coil body 20, a distal end joint part 30, and a proximal end fixing part 40.

The core shaft 10 has a round bar shape that tapers from a proximal end toward a distal end side. At an end portion on a proximal end side, a user performs rotational operation etc., of the guide wire 1.

The coil body 20 is formed in a hollow cylindrical shape by spirally winding a single metal wire 21 around the core shaft 10. As materials for the coil body 20, X-ray opaque materials such as gold, platinum, tungsten, tantalum or alloys containing these elements, or stainless steel, superelastic alloys, cobalt-based alloys, and the like can also be used.

The distal end joint part 30 constitutes a distal end of the guide wire 1 and has a substantially hemispherical shape. As materials for the distal end joint part 30, a metal such as silver, gold, or alloys of these metals, lead-free solder, brazing materials, adhesives, or the like is used. As examples of the lead-free solder and the brazing materials, lead-free solders, silver brazing materials, and gold brazing materials of Sn-Ag based alloy, Sn-Ag-Cu based alloy, Au-Sn based alloy, and Au-Ge based one can be used.

The proximal end joint part 40 fixes a proximal end of the coil body 20 to the core shaft 10. As materials for the proximal end joint part 40, a metal such as silver, gold, or alloys of these metals, lead-free solder, brazing materials, adhesives, or the like is used. As examples of the lead-free solder and the brazing materials, lead-free solders, silver brazing materials, and gold brazing materials of Sn-Ag based alloy, Sn-Ag-Cu based alloy, Au-Sn based alloy, and Au-Ge based one can be used.

The core shaft 10 is obtained by cutting the above-described medical wire material of the disclosure and tapering an end portion of the wire material so that the outer diameter gradually decreases toward the distal end. By using the medical wire material of the disclosure, a guide wire with excellent resilience can be obtained.

### [Examples]

Next, measurement of hardness, tensile strength measurement, and resilience evaluation of the wire material of the disclosure will be described. Tables 1 and 2 show the wire materials used in the evaluation. Table 1 shows the composition, wire diameter, tensile strength, and straightness of the wire drawing material used in Examples 1 to 5 and Comparative Examples 1 and 2.

**[Table 1]**

| | Chemical composition (mass%) | | | | | | | | Wire diameter (mm) | Tensile strength (N/mm²) |
|---|---|---|---|---|---|---|---|---|---|---|
| | Fe | Cr | Mn | Mo | Si | N | C | Ni | | |
| Wire drawing material 1 | Bal. | 17.2 | 11.7 | 3.04 | 0.40 | 0.51 | 0.19 | 0.02 | 0.343 | 2550 |
| Example1-5 | | | | | | | | | | |
| Comparative Example 1 (ASTM F 2581) | | | | | | | | | | |
| Wire drawing material 2 | Bal. | 18.02 | 1.25 | 0.56 | 0.40 | 0.04 | 0.08 | 8.07 | | 2677 |
| Comparative Example 2 (SUS 304) | | | | | | | | | | |

The wire drawing materials 1 used in Examples 1 to 5 and Comparative Example 1 shown in Table 1 are wire materials consisting of stainless steel, conforming to ASTM F2581. The wire drawing material 2 used in Comparative Example 2 is a wire material consisting of stainless steel of SUS304.

### Example 1

Plastic processing is performed on the wire drawing material 1 at a room temperature (25 degrees Celsius), then tension annealing is performed under a condition of heating to 500 to 650 degrees Celsius in a state where both ends of the wire material are gripped and a tensile force of 60N is applied, and thereby, the medical wire material was obtained.

### Example 2

The medical wire material was obtained in a similar manner to that of Example 1 except for a change in the heating temperature to 450 to 600 degrees Celsius.

### Example 3

The medical wire material was obtained in a similar manner to that of Example 1 except for a change in the heating temperature to 400 to 550 degrees Celsius.

### Example 4

The medical wire material was obtained in a similar manner to that of Example 1 except for a change in the heating temperature to 550 to 700 degrees Celsius.

### Example 5

The medical wire material was obtained in a similar manner to that of Example 1 except for a change in the condition of plastic processing at room temperature (25 degrees Celsius).

### Comparative Example 1

The medical wire material was obtained in a similar manner to that of Example 1 except for not performing tension annealing.

### Comparative Example 2

The medical wire material was obtained in a similar manner to that of Example 1 except for using the wire drawing material 2.

With respect to the wire materials obtained in Examples 1 to 5 and Comparative Examples 1 and 2, hardness and tensile strength were measured and resilience evaluation was performed.

### <Hardness Measurement>

The wire materials of Examples 1 to 5 and Comparative Examples 1 to 2 were embedded in a resin, polishing was performed such that a transverse section of the wire material is perpendicular to a measurement shaft (indenter shaft), and measurement of Nanoindentation hardness was performed on the polished transverse section under a condition of the maximum indentation load of 30 mN and the indentation interval of 0.01 mm by using a Berkovich indenter made of diamond of the nanoindentor (iMicro) manufactured by KLA Corporation on the basis of the Nanoindentation method (ISO 14577 "instrumented indentation test").

FIG. 2 is a schematic diagram illustrating a result of measuring a transverse section of a wire material by the Nanoindentation method. The transverse section of FIG. 2(a) indicates a measurement result of the wire material of Example 1 and the transverse section of FIG. 2(b) indicates a measurement result of the wire material of Comparative Example 2. Each of dots in each transverse section is a square of 0.01 mm square and the dots correspond to measurement portions. In addition, each of dots is classified by colors such that, as the measured hardness is greater, the dot is colored by a lighter color, and it can be seen that the wire material in Example 1 is entirely harder.

In order to exclude the influence of the resin used in embedding, the measurement points having hardness of less than 7.0 GPa were excluded.

### <Tensile Test>

The tensile strength of the wire materials of Examples 1 to 5 and Comparative Examples 1 and 2 was measured by a tensile test of a metal wire material (a tensile test conforming to JIS Z 2241 "metal material tensile testing method").

### <Resilience Evaluation>

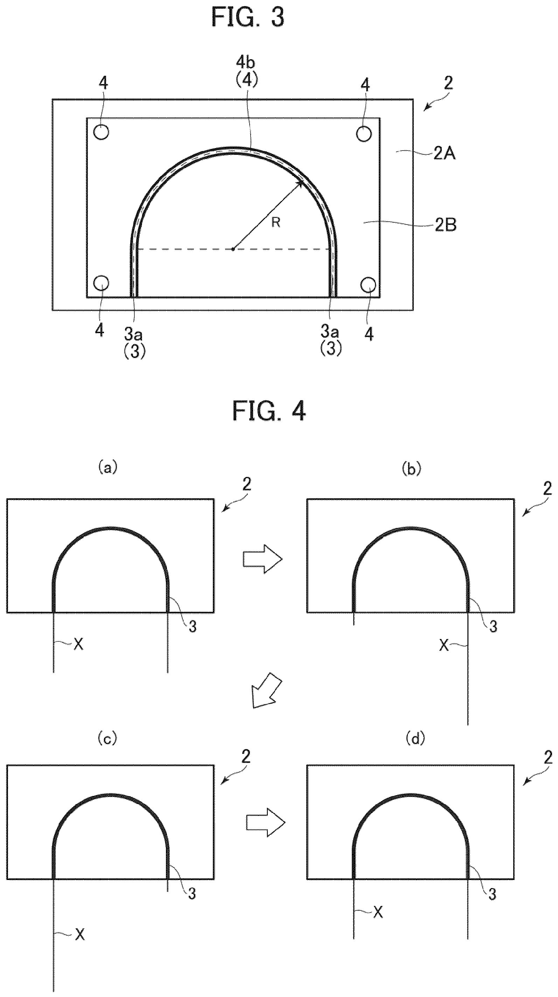

The residual angle of each wire material after the wire materials of Examples 1 to 5 and Comparative Examples 1 and 2 were inserted to a groove 3 of a jig 2 illustrated in FIG. 3.

FIG. 3 is a schematic diagram of the jig 2 to be used for resilience evaluation.

The first jig 2 is made of a resin (for example, a transparent acrylic plate) and is constituted by a first plate 2A having the groove 3 and a second plate 2B for covering the groove 3. The groove 3 of the first plate 2A is formed by a convex portion provided in the first plate 2A. The groove 3 includes a pair of straight portions 3a and a semicircular arc portion 3b. The width of the groove 3 is 1.0 mm and the depth of the groove 3 is 1.0 mm. The length of the straight portion 3a is 10 mm, and the radius R of the semicircular arc portion 3b is 10.0 mm. The second plate 2B covers the groove 3 in contact with the convex portion of the first plate 2A. The second plate 2B is fixed to the first plate 2A by four bolts 4.

FIG. 4 is an explanatory view of resilience evaluation.

In a state where the bolt 4 (FIG. 3) is loosened, as shown in FIG. 4(a), the wire material X cut to have a predetermined length (for example, 100 mm) is inserted to the groove 3 and the bolt 4 is tightened. As shown in FIG. 4(b), one end portion of the wire material X is pushed to a portion close to an opening of the groove 3 of the first jig 2. As shown in FIG. 4(c), the other end portion of the wire material X is pushed to a portion close to the opening of the groove 3 of the first jig 2. As shown in FIG. 4(d), one end portion of the wire material X is pushed, the center of the wire material X is moved to the center of the groove 3, the bolt 4 is loosened, and the wire material X is taken out. As a result, strain (about 1.7) is applied to the entire wire material X. The strain amount (%) is calculated by (R/(L-R))×100. Here, R is a wire diameter of the wire material X, and L is a diameter of the semicircular arc portion 3b of the groove 3 to which the wire material X is inserted.

As shown in FIG. 5, tangent lines are drawn from both ends of the wire material X, and the angle at which the two tangent lines intersect (residual angle θ) is measured.

Table 3 shows results of hardness measurement, tensile test, and resilience evaluation (residual angle).

**[Table 2]**

| | | Example 1 | Example 2 | Example 3 | Example 4 | Example 5 | Comparative example 1 | Comparative example 2 |
|---|---|---|---|---|---|---|---|---|
| Type of Wire drawing material | | Wire drawing material 1 (ASTM F2581) | Wire drawing material 1 (ASTM F2581) | Wire drawing material 1 (ASTM F2581) | Wire drawing material 1 (ASTM F2581) | Wire drawing material 1 (ASTM F2581) | Wire drawing material 1 (ASTM F2581) | Wire drawing material 2 (SUS 304) |
| Plastic deformation (room temperature) | | Performed | Performed | Performed | Performed | Performed | Performed | Performed |
| Tension annealing | | Performed | Performed | Performed | Performed | Performed | Not performed | Performed |
| Tensile test (N/mm²) | Tensile strength | 3146 | 3158 | 3106 | 2921 | 2986 | 2424 | 2848 |
| Residual angle (θ) | R10 | 6.5 | 7.0 | 11.0 | 9.5 | 11.9 | 41.5 | 18.9 |
| Hardness (GPa) | Ave (entire) | 9.48 | 9.57 | 9.37 | 9.09 | 8.94 | 7.89 | 8.79 |
| | Ave (outer peripheral portion) | 9.09 | 9.17 | 9.01 | 8.82 | 8.44 | 7.59 | 7.84 |
| | Ave(entire) - Ave (outer peripheral portion) | 0.39 | 0.40 | 0.36 | 0.27 | 0.50 | 0.30 | 0.95 |

diagram illustrating only dots of an outer peripheral portion C3 among all dots of the transverse section of the wire material illustrated in FIG. 2.

As the wire materials of Examples 1 to 5, straightening processing by plastic deformation is performed on a wire drawing material made of stable austenitic stainless steel, then tension annealing processing is performed, and thereby, average hardness of the outer peripheral portion of the transverse section is increased, so that a wire material having a small residual angle can be obtained. In addition, the average hardness of the entire transverse section of the wire material is increased, and a hardness difference between the average hardness of the entire transverse section of the wire material and the average hardness of the outer peripheral portion of the transverse section of the wire material is reduced.

On the other hand, in the wire material of Comparative Example 1, the tension annealing processing is not performed on the wire drawing material, so that the average hardness of the entire transverse section of the wire material is lower and the residual angle is larger. Since the plastic processing and tension annealing processing are performed on the wire drawing material consisting of SUS304, the average hardness of the entire transverse section of the wire material of Comparative Example 2 is equivalent to that of Example 5, while the residual angle is large due to lower hardness of the outer peripheral portion.

Note that the disclosure is not limited to the configuration of the above-described embodiments, but is defined by the terms of the claims and is intended to include any modifications within the scope and meaning equivalent to the terms of the claims.

For example, in the above embodiments, the medical wire material is the core shaft 10 of the guide wire 1, but the medical wire material may be a wire configuring the coil body 20, or a wire material to be used in other medical instruments, such as a wire that constitutes a reinforcing body (for example, a tubular mesh body, a coil body) to be used for reinforcing a catheter, for example. The coil body 20 or the reinforcing body of a catheter is constituted by using the medical wire material of the above embodiments, so that it is possible to improve the resilience of the coil body 20 or the reinforcing body after bending. In addition, medical members including coils or hollow bodies produced using the wire material become possible to exhibit excellent rotational followability in the same manner as described above when rotational operation is performed in intricate blood vessels.

### [Reference Signs List]

- 1: guide wire
- 10: core shaft
- 20: coil body

## Claims

1. A medical wire material comprising stainless steel, wherein
a shape of a transverse section of the wire material is a circle having a diameter of d mm, and,
when hardness of the transverse section of the wire material is measured by the Nanoindentation method, an average value of hardness of an outer peripheral portion constituted of a region surrounded by an outer peripheral edge and a circle having a distance from the outer peripheral edge is d/17 mm is equal to or greater than 8.0 GPa.

2. The medical wire material according to claim 1, wherein the stainless steel is stable austenitic stainless steel having a temperature Md₃₀ that is equal to or lower than - 50 degrees Celsius, the temperature Md₃₀ being the temperature at which strain-induced martensite of 50% occurs when a strain of 30% given by an expression of Angel is applied.

3. The medical wire material according to claim 2, wherein the stable austenitic stainless steel is stainless steel conforming to ASTM F2581.

4. The medical wire material according to any one of claims 1 to 3, wherein, when the hardness of the transverse section of the wire material is measured by the Nanoindentation method, a difference between an average value of hardness of an entirety of the transverse section of the wire material and an average value of hardness of the outer peripheral portion is equal to or less than 0.7 GPa.

5. The medical wire material according to any one of claims 1 to 4, wherein an average value of hardness of an entirety of the transverse section of the wire material is equal to or greater than 8.9 GPa.

6. A guide wire comprising the medical wire material according to any one of claims 1 to 5.
